# FASCICULE DE BREVET EUROPEEN

(11) **EP 2 915 508 B1**
(45) Date de publication et mention de la délivrance du brevet: **23.05.2018**
(21) Numéro de dépôt: 15157853.1
(22) Date de dépôt: 05.03.2015
(51) Int. Cl.: A61F 2/46, A61F 2/30

(54) **DISPOSITIF IMPACTEUR POUR UN ÉLÉMENT INSERT**
IMPAKTORVORRICHTUNG FÜR EIN EINSATZELEMENT
IMPACTOR DEVICE FOR AN INSERT MEMBER

(30) Priorité: 07.03.2014 FR 1400563
(43) Date de publication de la demande: 09.09.2015
(73) Titulaire: XNOV IP, 1882 Luxembourg (LU)
(72) Inventeur: Biegun, Jean-François, 2900 Porrentruy (CH); Biegun, Frédérique, 2900 Porrentruy (CH); Loehle, Pascal, 2900 Porrentruy (CH)
(74) Mandataire: Eidelsberg, Olivier Nathan

(56) Documents cités:
- EP-A1- 0 931 523
- EP-A1- 1 190 687
- EP-A1- 1 707 160
- EP-A1- 2 422 754
- EP-A2- 1 666 007
- WO-A1-2010/109327
- DE-U1-202006 000 845
- US-A- 5 061 270
- US-A- 5 735 855
- US-A1- 2013 204 264

## Description

La présente invention se rapporte à un dispositif dit « impacteur » pour « impacter » une tige, c'est à dire lui impartir un choc, la tige étant destinée à transmettre le choc à un élément insert pour insérer celui ci dans un élément de réception, par exemple un élément insert, notamment en matériau céramique, à l'intérieur d'une cupule elle-même destinée à être reçue dans la cavité cotyloïdienne d'une hanche d'un patient. La présente invention se rapporte également à un assemblage comportant un dispositif impacteur de ce genre et au moins une tige destinée à transmettre le choc à un élément insert.

On connaît déjà par exemple de EP1707160 ou de WO 2010/109327 un dispositif impacteur destiné à impacter avec une force ou puissance prédéterminée à l'avance une tête sphérique de manière à ce qu'elle coiffe une tige, l'application du choc ayant lieu après positionnement de la tête au dessus de la tige avec cette dernière légèrement introduite dans la cavité de la tête destinée à la recevoir.

De DE 20 2006 000845, on connaît un dispositif impacteur comportant un canon 1, dans lequel un piston percuteur 3 monté sur ressort se déplace pour impacter l'extrémité d'une tige 8 fixe par rapport au canon, qui elle-même transmet le choc à un élément d'une prothèse en vue de réaliser une liaison conique entre l'élément impacté et un autre élément.

De EP 1 666 007, on connaît un dispositif pour insérer une cupule acétabulaire souple dans une cavité hôte de l'os.

Ces dispositifs impacteur de l'art antérieur ne permettent pas d'effectuer un impactage précis de la tige qui transmet le choc à l'insert, et il est fréquent que l'insert soit mal inséré dans l'élément de réception ou soit détérioré, notamment fêlé, voire brisé, notamment dans le cas de l'utilisation de ces impacteurs pour insérer un insert en céramique dans une cupule pour cotyle de hanche.

La présente invention vise à surmonter les inconvénients de l'art antérieur en proposant un dispositif impacteur d'une tige destinée à transmettre le choc à un élément insert à insérer dans un élément de réception qui permet à coup sûr que l'insert soit convenablement insérer dans l'élément de réception, notamment la cupule, sans le détériorer, notamment sans le fêler, le casser ou le mettre de travers.

Suivant l'invention, un dispositif impacteur est tel que défini à la revendication 1.

Suivant l'invention, le chirurgien peut prendre le dispositif impacteur à pleine main, c'est à dire avec au moins les quatre doigts d'une main en contact avec la crosse, d'un seul et même côté, en dessous du canon, pour ainsi pouvoir facilement orienter le canon en visant avec son oeil sans que dernier soit obstrué, notamment par les moyens de prise à la main. En outre, il peut dans le même temps avoir son avant bras en ligne avec le dos de la paume de la main pour ainsi pouvoir appliquer une grande pression du canon sur la tige.

En prévoyant ainsi des moyens de prise à la main du dispositif impacteur qui permettent au chirurgien non seulement de bien orienter l'axe du canon et donc l'axe de la tige par rapport à l'insert mais aussi d'appliquer une grande force de poussée du canon contre la tige pour éviter toute déperdition de puissance à l'interface entre le piston et la tige, on s'assure que la force ou puissance théorique déterminée à l'avance aussi bien en amplitude qu'en direction est bien appliquée à la tige et à l'insert. On évite ainsi que le choc transmis à l'insert soit insuffisant pour sa bonne insertion dans la cupule, le choc réel imparti à l'insert dans les dispositifs antérieurs étant toujours inférieur au choc théorique en raison des déperditions au niveau de l'interface impacteur - tige et du mauvais alignement de l'impacteur et/ou de la tige par rapport à l'insert.

Des perfectionnements sont définis aux sous revendications 2 à 9.

La présente invention se rapporte aussi à un assemblage suivant l'une des revendications 10 à 14.

En prévoyant des moyens pour tenir l'insert en même temps qu'il est impacté, on est en mesure, en fonction des données et/ou de l'expérience, d'adapter la puissance du choc pour optimiser le coup donné à l'insert pour l'insérer. L'insert n'est pas susceptible, lors du choc, de subir une légère rotation, de sorte que la valeur effective préréglée de la force d'impact est parfaitement répercutée et donc reproduite et reproductible, contrairement à ce qui est prévu dans l'art antérieur, par exemple notamment dans EP 1190687 dans lequel le non maintien par des moyens de maintien lors de l'application du choc fait que l'axe d'application du choc ou impact peut varier d'un choc à l'autre et par conséquent nuire à la reproductibilité de l'impact, qui en théorie est de valeur constante mais en pratique ne l'est pas. Dans EP 1 190 687, cet inconvénient est aggravé par le fait que le choc est appliqué sur la tête sphérique recevant l'insert et non sur l'insert. On évite ainsi des variations de la puissance, soit en fonction du praticien et/ou de sa forme du jour, soit en raison d'un mouvement concomitant de l'insert impacté, susceptible d'entraîner soit une fêlure ou cassure de l'insert ou de la cupule, soit une insertion incomplète de l'insert dans la cupule.

On obtient ainsi une insertion par impaction réalisée d'une manière particulièrement fiable et simple, en diminuant dans une très grande mesure le risque d'une insertion « désaxée », source potentielle de rupture ou fêlure de la cupule une fois posée dans le cotyle. Quelque soit l'angle de la force réalisant l'impaction par rapport à l'insert, c'est à dire l'orientation de la tige par rapport à l'insert, ce dernier étant maintenu par le dispositif d'insertion avec possibilité de pivotement se positionne toujours parfaitement par rapport à la cupule, quelque soit l'orientation de la tige par rapport à la cupule, de sorte que le chirurgien n'a plus besoin, comme dans l'art antérieur, de s'assurer que la tige arrive parfaitement perpendiculaire au plan de base de la cupule lors de l'insertion.

De préférence, le point pivot du montage pivotant des moyens de préhension par rapport à l'ensemble tige-tête d'impaction se trouve sensiblement au centre de la sphère intérieure de l'insert.

Suivant un mode de réalisation préféré de l'invention, les moyens de préhension de l'insert sont constitués d'une griffe de maintien comportant au moins deux, de préférence trois, pattes destinées à enserrer entre elles l'insert de manière libérable élastiquement.

De préférence, la griffe de maintien comporte une plaque de base de la périphérie de laquelle font saillie les pattes.

En particulier, la plaque de base est percée d'un trou, notamment sensiblement central, dont la surface de la paroi intérieure épouse la forme d'une rotule formée sur la tige de manière à permettre le pivotement relatif de la tige et de la plaque de base.

Suivant un mode de réalisation avantageux, l'extrémité distale de la tige comporte une tête faisant saillie latéralement de la tige et constituant la tête d'impact, la tête ayant une surface d'extrémité distale, notamment en forme de dôme, destinée à épouser en partie la forme du fond de la cavité intérieure de l'insert.

La présente invention se rapporte également à un ensemble tel que défini à la revendication 15.

Suivant un perfectionnement constituant également une invention en tant que tel, indépendamment de l'invention décrite ci dessus, il est prévu des moyens pour impartir à la tige un choc vers le bas pour impacter l'insert dans la cupule, c'est à dire l'introduire à adaptation en force à l'intérieur de la cupule, ces moyens pour impartir impartissant à la tige un choc dont la puissance a une valeur déterminée à l'avance.

Suivant un mode de réalisation préféré de l'invention, les moyens pour impartir comportent un piston et un ressort.

De préférence les moyens pour impartir comportent des moyens pour déplacer le piston à l'encontre de la compression du ressort de manière à comprimer le ressort et des moyens pour bloquer le piston en une position dans laquelle le ressort est comprimé et des moyens, notamment sous la forme d'une détente, destinés à libérer le piston, la libération du piston entraînant son déplacement instantané par la décompression du ressort et son action déterminée à l'avance sur la tige impartissant à cette dernière le choc de puissance déterminée à l'avance.

De préférence, le ressort et le piston sont reçus dans une cavité formée en amont de la tige.

De préférence, la cavité recevant le piston et le ressort est formée dans un châssis disposé en amont de la tige, notamment un châssis d'une pièce avec la tige.

De préférence une poignée, notamment de type crosse à pistolet, fait saillie du châssis.

On décrit maintenant, à titre d'exemple, des modes de réalisation de l'invention en se reportant aux dessins, dans lesquels :
• la figure 1 représente un dispositif d'insertion suivant l'invention, une cupule et un insert en céramique destiné à être inséré par le dispositif d'insertion à l'intérieur de la cupule 50;
• les figures 2A, 2B et 2C représentent les différentes étapes de l'insertion de l'insert à l'aide du dispositif d'insertion dans la cupule ;
• la figure 3 est une vue en perspective de dessous d'une partie du dispositif d'insertion de la figure 1 ;
• la figure 4 est une vue en coupe d'un dispositif d'impactage ou d'application d'un choc suivant un mode de réalisation de l'invention, qui peut notamment être utilisé pour impacter la tige du dispositif d'insertion des figures précédentes; et
la figure 5 représente le dispositif d'impactage de la figure 4 tel qu'utilisé par un chirurgien, la tige insérée par l'ouverture distale pouvant notamment être celle du dispositif décrit aux figures 1 à 3.

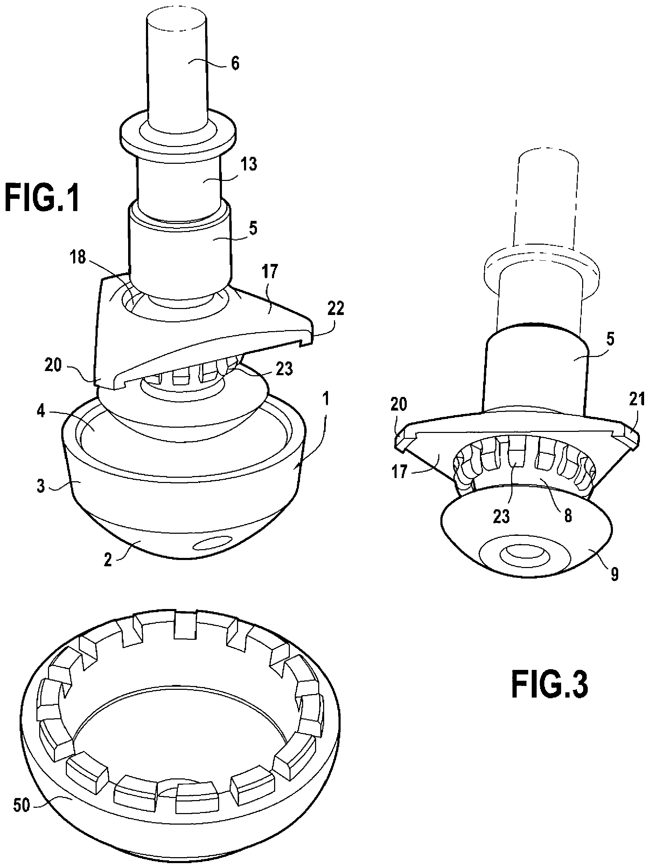

A la figure 1, il est représenté suivant une vue en perspective un dispositif d'insertion suivant un mode de réalisation de l'invention d'un insert 1 en matériau céramique dans une cupule 50. L'insert 1 en céramique est constitué d'un corps en céramique, notamment en céramique d'alumine ayant une surface extérieure en forme de calotte 2 sphérique terminée par une partie 3 de bord supérieur ou proximal de forme légèrement tronconique. L'insert est creux et comporte une cavité 4 intérieure dont la paroi est de forme hémisphérique. En particulier, le cône inscrit de la partie tronconique 3 de bord de la surface extérieure de l'insert fait un angle d'environ 3 à 5° par rapport à l'axe vertical de symétrie de l'insert en céramique.

Le dispositif d'insertion est constitué d'une tige 6 cylindrique circulaire à laquelle a été adapté à son extrémité distale un embout 5 d'impaction. L'embout 5 comporte un tronçon proximal tubulaire 7 taraudé pour recevoir en son sein l'extrémité filetée de la tige 6, pour ainsi les solidariser par vissage. On pourrait, à la place, prévoir que l'embout soit maintenu avec la tige à l'aide d'un autre type de liaison, par exemple un système d'encliquetage. On pourrait aussi prévoir que l'embout soit monobloc avec la tige.

L'embout 5 comporte ensuite un tronçon intermédiaire en forme de bille 8 sphérique, puis un tronçon distal formant tête 9 d'impaction. La tête 9 d'impaction est de plus grande dimension transversale que le reste de l'embout 5, notamment que le tronçon 7 proximal, et sa surface inférieure ou distale est en forme de dôme en étant au moins en partie complémentaire de la surface intérieure de la cavité 4 de l'insert 1, de manière à pouvoir réaliser avec celle ci en contact de surface. On pourrait prévoir cependant, à l'inverse que La tête 9 d'impaction soit de plus petite dimension transversale que le reste de l'embout 5, notamment que le tronçon 7 proximal.

La surface extérieure de la tête 9 destinée à s'appliquer contre la paroi de fond de la cavité 4 est inscrite dans une sphère, concentrique avec la sphère définissant la bille 8. Cela facilite la libre orientation de l'insert.

A l'intérieur de l'embout 5, il est formé au niveau de la fin du tronçon 7 proximal, une butée 10 contre laquelle vient buter l'extrémité distale de la tige 6, pour en bloquer son vissage avec le taraudage interne du tronçon 7. En outre, un canal 11 débouchant à la surface inférieure en forme de dôme du tronçon 9 par une ouverture facilitant le nettoyage interne de l'embout.

Un élément 13 de raccordement également tubulaire termine la liaison entre l'embout 5 et la tige 6. Cet élément 13 comporte une extrémité taraudée qui se visse dans la partie du filetage de la vis qui ne coopère pas avec le taraudage de l'embout 5. De l'autre côté, proximal, l'élément 13 est soudé en 14 à la tige.

Un élément formant griffe est monté pivotant par rapport à la bille 8. Cette griffe comporte une plaque 17 sensiblement plane, de forme triangulaire et aux trois sommets de laquelle font saillies trois pattes 20, 21 et 22 respectives. Les pattes sont destinées à enserrer l'insert au niveau de son bord supérieur ou proximal. Les pattes et/ou la plaque sont en un matériau et/ou ont une épaisseur tels qu'elles présentent une certaine élasticité leur permettant d'enserrer élastiquement le bord supérieur de l'insert 1 de manière élastique et de le relâcher lorsque qu'une force de déformation est appliquée à la griffe, par exemple en appuyant sur la plaque 17. La plaque 17 est percée en son milieu d'un trou 18 de forme complémentaire de la bille 8 sphérique formant rotule et comporte des éléments 23 en forme de dents sensiblement de forme complémentaire de celle de la bille 8 sphérique de manière à en épouser la forme. Le matériau et/ou l'épaisseur des dents est (sont) choisi(s) de manière à permettre l'encliquetage élastique de la plaque sur la rotule. le bord intérieur du trou 18 a une paroi intérieure de forme incurvée complémentaire de la forme de la bille 8 formant rotule. Le montage est tel que la plaque 17, une fois encliquetée par les dents 23 à la rotule 8, peut pivoter par rapport à la rotule 8, et donc par rapport à la tige 6 solidaire, notamment en rotation, de la rotule 8.

Dans le mode de réalisation représenté, on a prévu trois pattes. Cependant, on pourrait en prévoir plus, notamment quatre ou cinq pour des inserts de grande taille. On pourrait aussi à la place des pattes prévoir une galette faisant tout le tour.

La dimension de la plaque est réalisée de telle manière que lorsque la tête 9 vient en contact contre la surface de la paroi intérieure de l'insert en céramique, les trois pattes 20, 21 et 22 enserrent le bord périphérique libre de la partie 3 tronconique de l'insert 1 en céramique, les trois pattes 20, 21 et 22 s'étendant alors vers la cupule le long de la surface extérieure de la partie 3 de l'insert 1 en céramique en enserrant ce dernier. L'insert 1 en céramique est ainsi pris par la griffe de manière élastique.

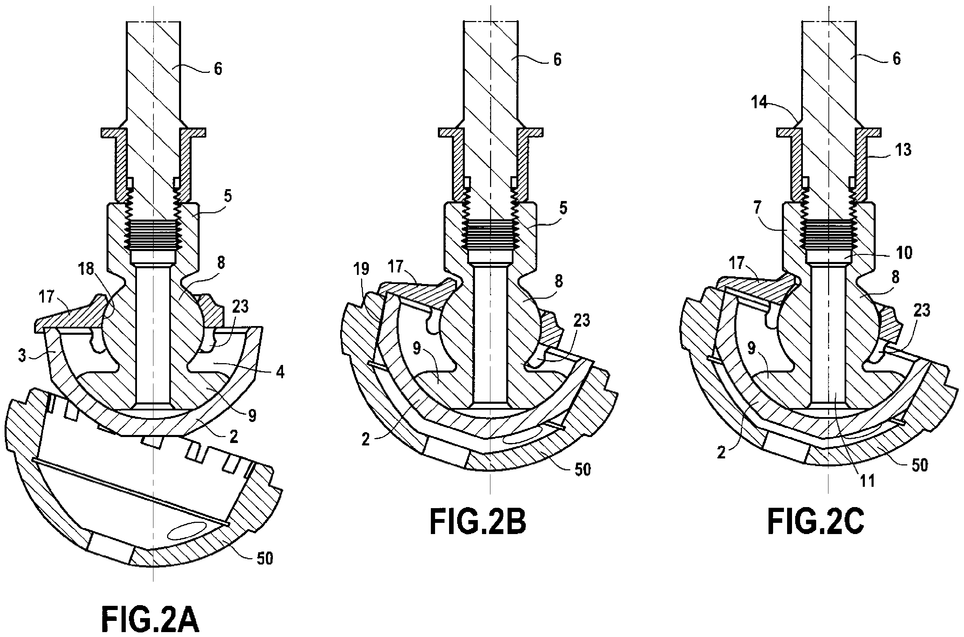

Une fois l'insert pris entre les pattes 20, 21, 22 (Figure 2A), on positionne à l'aide du dispositif l'insert 1 à l'intérieur de la cupule (Figure 2B). Cette étape se fait sans qu'il soit besoin que l'axe d'insertion (l'axe de la tige) soit perpendiculaire au plan de base ou supérieur de la cupule ou de l'insert, grâce au montage en pivotement relatif de l'ensemble griffe-insert préalablement solidarisé mutuellement par serrage des pattes. Cette deuxième étape de poussée a lieu jusqu'à ce que les bords distaux des pattes 20, 21 et 22 viennent buter contre le bord libre supérieur de la cupule. A ce moment, l'insert est en grande partie introduit dans la cupule en ayant sa partie 3 tronconique presque en contact avec la face interne de la cupule, dans une section 19 haute ayant une forme complémentaire de cet élément tronconique de l'insert. Les deux parties tronconiques de l'insert et de la cupule sont donc maintenues dans une position coaxiale mais sans solidarisation puisque les pattes maintiennent un jeu minimum entre l'insert et la cupule. Il en résulte que la simple poussée de l'insert dans la cupule en vient à être arrêtée à peu près au même moment que les extrémités distales des pattes 20, 21, 22 viennent en contact avec le bord libre de la cupule.

Ensuite, pour insérer de force l'insert dans la cupule, le chirurgien va appliquer un choc sur la tige, par exemple en donnant un coup sur le dessus de la tige vers le bas, dans la direction d'extension de la tige. Compte tenu de la souplesse des pattes et/ou de la plaque, ce choc va simultanément enfoncer de force l'insert dans sa position finale dans la cupule (position représentée à la figure 2C) et libérer les pattes de leur emprise sur l'insert.

Comme l'insert a été positionné grâce au dispositif de l'invention, parfaitement centré par rapport à la cupule dans la position de la figure 2B avant application du choc, il n'est pas nécessaire que la tige soit parfaitement perpendiculaire au plan supérieur de la cupule et de l'insert (plans dans lesquels s'étendent respectivement les bords libres de la cupule et de l'insert) lors de l'application de ce choc, l'angle entre la tige et l'axe de l'insert peut en particulier, comme représenté à la figure 2C, avoir une valeur élevée, par exemple 10 à 20°, sans que cela n'ait la moindre incidence sur la « qualité » de l'insertion finale à force de l'insert.

Le blocage relatif de l'insert dans la cupule est réalisé par la légère différence de conicité des parois intérieure 19 de la cupule et 3 extérieure de l'insert 1. Cependant, on pourrait prévoir d'autres manière de réaliser cette adaptation en force, par exemple en prévoyant des saillies de la surface extérieure de l'insert.

Ci dessus, on a décrit un premier mode de réalisation dans lequel le chirurgien applique le choc d'impaction en donnant un coup sur la tige.

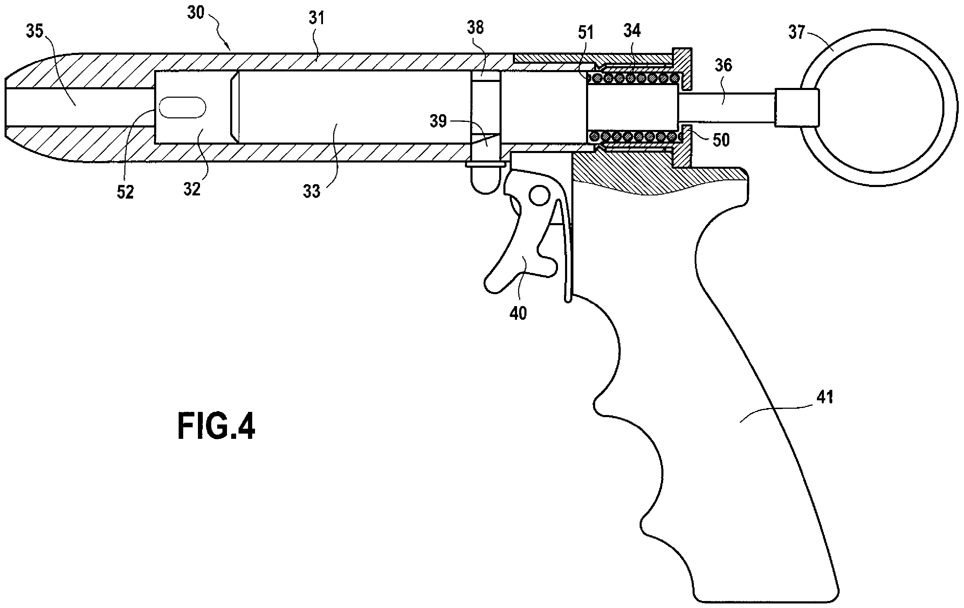

On peut, suivant un autre mode de réalisation avantageux, prévoir à l'extrémité proximale de la tige 6 une partie en forme de pistolet. Cette partie en forme de pistolet comporte un châssis 30 comportant un canon 31 destiné à être relié, notamment de manière solidaire, à la tige 6. Dans le canon 31, il est formé une cavité 32 dans laquelle est monté mobile en translation un piston 33 le long de l'axe du canon. Un ressort 34 est disposé à l'arrière du piston. Une tige 36 solidaire du piston 33 passe à travers le ressort et sort du châssis 30 par une ouverture 50 arrière. La tige 36 permet à l'utilisateur, notamment par l'intermédiaire d'un anneau 37 solidaire de la tige 36, de tirer le piston 33 vers l'arrière en comprimant ainsi le ressort 34 contre un épaulement 51 du piston 33 jusqu'à ce qu'une encoche circulaire 38 transversale vienne se bloquer par encliquetage dans une butée 39. Lorsque la butée 39 est bloquée dans l'encoche 38, le piston est déplacé le plus en arrière possible et le ressort 34 est comprimé. La butée 39 est montée rotative, de sorte qu'une queue de détente 40, montée rotative, entraîne la rotation de la butée 39 et donc sa sortie de l'encoche 38 et in fine la libération du piston 33. Sous l'effet de la charge du ressort pré-comprimé, le piston 33 se déplace alors à grande vitesse vers la gauche à la figure 4 et vient frapper l'extrémité distale de la cavité 32, entrant en collision avec la tige 6, dont l'extrémité proximale a été préalablement introduite dans un canal 35 d'introduction jusqu'au niveau de l'ouverture 52 distale de la cavité 32. Des moyens d'arrêt de l'introduction de la tige dans le canon 31 peuvent être prévus pour que la tige pénètre dans le canal 35 mais s'arrête à l'entrée 52 de la cavité 32. Ces moyens d'arrêt peuvent par exemple être une butée 60 en forme de bague circulaire faisant saillie latéralement de la tige et venant buter contre l'ouverture distale de sortie du canal 35.

Le dispositif comporte également une poignée 41 en forme de crosse de pistolet.

La tige subit alors un choc qui impacte l'insert en céramique pour l'insérer dans la cupule.

Ainsi, la force d'impaction appliquée à la tige 6 peut être déterminée à l'avance par le choix du ressort 34, de sa raideur et de la course de déplacement vers l'arrière du piston 33. On s'assure ainsi d'une force d'impulsion constante sur la tige et par conséquent d'une force ou puissance d'impaction de l'insert dans la cupule qui est constante ou sensiblement constante, correspondant exactement à ce qui est nécessité pour assurer une bonne insertion de l'insert, sans pour autant que ce dernier ne soit fêlé, comme c'était le cas dans l'art antérieur où il était difficile de doser l'effort du marteau. La vitesse impartie à la tige par la libération du piston peut être déterminée à l'avance par le choix de la géométrie des éléments constitutifs du dispositif d'insertion, notamment du ressort, du piston, de la longueur du tube 6 et analogue.

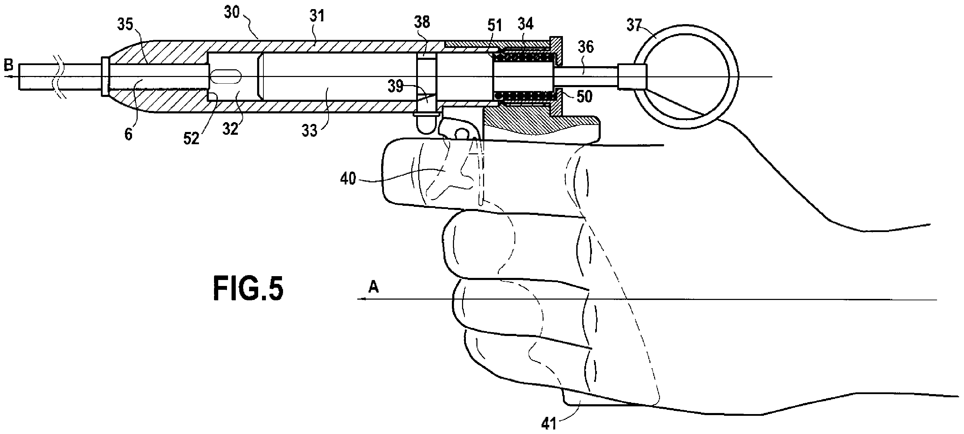

A la figure 5, on voit comment est utilisé le dispositif impacteur de la figure 4. Le chirurgien prend le dispositif à la main en empoignant la crosse 41 qui a une forme et une dimension telle que lorsque le chirurgien la tient tel que prévu normalement, à la manière d'un pistolet, son avant bras et le dos de la main sont alignés mutuellement, sans que le poignet soit cassé et l'axe A de l'avant bras est sensiblement parallèle à l'axe B longitudinal du canon. En outre, les quatre doigts de la main peuvent être en contact simultanément avec la crosse en étant tous du même côté du canon, c'est à dire sous le canon lorsque ce dernier pointe à l'horizontale.

Le chirurgien peut presser le canon du pistolet fermement contre la tige à laquelle il entend impartir un choc pour assurer un positionnement parfait de celle ci par rapport au piston pour que ce dernier lui délivre un choc tel que prévu aussi en bien en amplitude qu'en direction, évitant ainsi des déperditions à l'interface tige-piston au moment de l'impact, et donc un choc transmis à la tige moindre que prévu. En outre le chirurgien a plus de facilité pour bien viser à quel endroit appliquer le choc.

Dans la présente demande, on a décrit le dispositif de préhension et d'insertion de l'insert par le mode de réalisation des figures 1 à 3. Cependant, l'utilisation du dispositif d'impaction automatique des figures 4 et 5 n'est pas liée à ce mode de réalisation et on peut en particulier l'utiliser avec tout autre type de dispositif de préhension et d'insertion, par exemple un dispositif ne comportant qu'une simple tige, solidarisée ou non à l'insert, sans possibilité de pivotement mutuel, par exemple avec les dispositifs décrits dans US-A-4,994,064, EP-A-0931523, US-A-5,462,548 ou analogues, sans pour autant sortir de l'étendue de protection de l'invention conférée par la présente demande.

## Revendications

1. Dispositif impacteur pour impartir un choc à une tige (6) ou « impacter » une tige (6) destinée à transmettre le choc à un élément insert pour insérer celui ci dans un élément de réception, par exemple un élément insert, notamment en matériau céramique, à l'intérieur d'une cupule elle-même destinée à être reçue dans la cavité cotyloïdienne d'une hanche d'un patient, comportant un châssis (30) formant canon (31) définissant un canal intérieur (35) ayant un axe longitudinal et débouchant par une ouverture d'extrémité distale par laquelle peut être insérée au moins une extrémité proximale de la tige, des moyens d'impact destiné à impartir à l'extrémité proximale de la tige insérée dans le canal (35) un choc ayant une puissance déterminée à l'avance et des moyens de prise à la main destinés à la prise du dispositif impacteur par au moins une main du chirurgien, **caractérisé en ce que** les moyens de prise à la main sont réalisés en forme de crosse (41) de pistolet de sorte que le chirurgien puisse prendre le dispositif impacteur à pleine main, c'est à dire avec au moins les quatre doigts d'une main en contact avec la crosse, d'un seul et même côté, en dessous du canon.

2. Dispositif suivant la revendication 1, **caractérisé en ce qu'**il est prévu des moyens de butée de sorte que pendant l'application du choc à la tige par les moyens d'impact, le châssis formant canon, sous une force exercée par l'utilisateur tenant l'impacteur par la crosse, exerce aussi une pression sur la tige pour la presser contre l'insert.

3. Dispositif suivant l'une des revendications précédentes, **caractérisé en ce que** l'agencement est tel que seul un tronçon d'extrémité de la tige peut pénétrer dans le canal, les moyens de butée empêchant la tige de pénétrer au delà du tronçon.

4. Dispositif suivant l'une des revendications 1 à 3, **caractérisé en ce que** les moyens d'impact pour impartir un choc de puissance déterminée à l'avance à la tige comportent un piston et un ressort.

5. Dispositif suivant la revendication 4, **caractérisé en ce qu'**il est prévu des moyens pour déplacer le piston à l'encontre de la compression du ressort de manière à comprimer le ressort.

6. Dispositif suivant l'une des revendications précédentes, **caractérisé en ce qu'**il est prévu des moyens formant détente pour déclencher les moyens d'impact.

7. Dispositif suivant la revendication 5, **caractérisé en ce que** l'agencement est tel que le chirurgien peut déclencher les moyens d'impact en agissant sur une détente avec un doigt de la main, notamment l'index, tout en tenant le dispositif par les moyens de prise à la main en ayant son avant bras d'une part en ligne avec le dos de la paume de la main et d'autre part parallèle à l'axe longitudinal du canal.

8. Dispositif suivant la revendication 4, **caractérisé en ce que** sont prévus des moyens pour bloquer le piston dans une position chargée dans laquelle le ressort est comprimé et des moyens, notamment sous la forme d'une détente, destinés à libérer le piston, la libération du piston entraînant son déplacement instantané par la décompression du ressort et son action d'impulsion sur la tige impartissant à cette dernière un choc de puissance déterminée à l'avance.

9. Dispositif suivant la revendication 4, **caractérisé en ce que** le ressort et le piston sont reçus dans une cavité proximale à la tige, le tronçon de la tige introduit dans le canal étant maintenu par les moyens formant butée à distance du piston dans la position chargée.

10. Assemblage comportant un dispositif suivant l'une des revendications précédentes et une tige dont une extrémité peut être insérée dans le canal tubulaire du canon par l'ouverture distale.

11. Assemblage suivant la revendication 10, **caractérisé par** des moyens (17, 20, 21, 22) de préhension de l'insert, notamment au niveau d'un bord libre supérieur de l'insert, la tige (5,6) étant destinée à réaliser une impaction sur l'insert lorsqu'il est tenu par les moyens de préhension en face de la partie creuse de l'élément de réception, notamment la cupule.

12. Assemblage suivant l'une des revendications 10 ou 11, **caractérisé en ce que** il comporte une tête d'impaction ayant une surface destinée à venir en contact avec au moins une partie de l'insert, notamment la surface intérieure d'un creux de l'insert, et la tête d'impaction et la tige d'application sont liées l'une à l'autre d'une manière solidaire en rotation, notamment la tête est issue de la tige et l'ensemble tête d'impaction et tige est monté pivotant par rapport aux moyens de préhension de l'insert, notamment à la manière d'une rotule.

13. Assemblage suivant l'une des revendications 10 à 12, caractérisé pas des moyens formant butée empêchant la tige de pénétrer dans le canon au delà d'un tronçon proximal de la tige.

14. Assemblage suivant la revendication 13, **caractérisé en ce que** les moyens formant butée sont constitués d'un bague circulaire faisant saillie latéralement de la tige.

15. Ensemble comportant un assemblage suivant l'une des revendications 10 à 14 et au moins un insert (1), notamment en céramique, destiné à être inséré dans une cupule cotyloïdienne.

## Patentansprüche

1. Impaktor-Vorrichtung zum Ausüben eines Stoßes auf eine Stange (6) oder zum "Beaufschlagen" einer Stange (6) zur Übertragung des Stoßes auf ein Einsatzteil zum Einsetzen desselben in ein Aufnahmeteil, beispielsweise ein Einsatzteil, insbesondere aus keramischem Material, in einer Pfanne, die selbst dazu ausgelegt ist, in der Hüftgelenkspfanne einer Hüfte eines Patienten aufgenommen zu werden, umfassend einen Rahmen (30), der einen Lauf (31) ausbildet, der einen Innenkanal (35) mit einer Längsachse begrenzt und am distalen Ende in eine Öffnung mündet, durch die mindestens ein proximales Ende der Stange einführbar ist, ein Beaufschlagungsmittel zum Ausüben eines Stoßes mit einer vorab bestimmten Kraft auf das proximale Ende der in den Kanal eingeführten Stange (35) und ein Handgreifmittel zum Greifen der Impaktorvorrichtung durch mindestens eine Hand des Chirurgen, **dadurch gekennzeichnet, dass** das Handgreifmittel in Form eines Pistolengriffs (41) gestaltet sind, so dass der Chirurg die Impaktorvorrichtung mit der gesamten Hand ergreifen kann, d. h mit mindestens den vier Fingern einer Hand, die den Griff auf ein und derselben Seite unterhalb des Laufs berühren.

2. Vorrichtung nach Anspruch 1, **dadurch gekennzeichnet, dass** ein Anschlagmittel derart vorgesehen ist, dass während des Ausübens des Stoßes auf die Stange durch das Beaufschlagungsmittel der den Lauf bildende Rahmen aufgrund einer Kraft, die von dem Benutzer ausgeübt wird, der den Impaktor am Griff hält, ebenfalls einen Druck auf die Stange ausgeübt, um diese gegen den Einsatz zu pressen.

3. Vorrichtung nach einem der vorhergehenden Ansprüche, **dadurch gekennzeichnet, dass** die Anordnung derart ist, dass nur ein Endstück der Stange in den Kanal eindringen kann, wobei die Anschlagmittel verhindern, dass die Stange über das Endstück hinaus eindringt.

4. Vorrichtung nach einem der Ansprüche 1 bis 3, **dadurch gekennzeichnet, dass** die Beaufschlagungsmittel zum Ausüben eines Stoßes mit vorbestimmter Kraft auf die Stange einen Kolben und eine Feder umfassen.

5. Vorrichtung nach Anspruch 4, **dadurch gekennzeichnet, dass** Mittel vorgesehen sind, um den Kolben gegen den Druck der Feder zu bewegen, so dass die Feder zusammengedrückt wird.

6. Vorrichtung nach einem der vorhergehenden Ansprüche, **dadurch gekennzeichnet, dass** ein einen Abzug bildendes Mittel zum Auslösen des Beaufschlagungsmittels vorgesehen ist.

7. Vorrichtung nach Anspruch 5, **dadurch gekennzeichnet, dass** die Anordnung derart ist, dass der Chirurg das Beaufschlagungsmittel durch Einwirken auf einen Abzug mit einem Finger der Hand, insbesondere dem Zeigefinger, auslösen kann, während er die Vorrichtung an der Handgreifeinrichtung erfasst hat und dabei einerseits seinen Unterarm mit dem Handrücken in einer Linie und andererseits parallel zur Längsachse des Kanals hält.

8. Vorrichtung nach Anspruch 4, **dadurch gekennzeichnet, dass** ein Mittel zum Arretieren des Kolbens in einer belasteten Stellung, in der die Feder zusammengedrückt ist, und ein Mittel, insbesondere in Form eines Abzugs, zum Freigeben des Kolbens vorgesehen sind, wobei das Freigeben des Kolbens seine sofortige Verschiebung durch die Entspannung der Feder und eine Impulswirkung des Kolbens auf die Stange bewirkt, wodurch auf diese ein Stoß mit vorbestimmter Kraft ausgeübt wird.

9. Vorrichtung nach Anspruch 4, **dadurch gekennzeichnet, dass** die Feder und der Kolben in einem zur Stange proximalen Hohlraum aufgenommen sind, wobei das in den Kanal eingeführte Endstück der Stange durch das einen Anschlag bildende Mittel in einem Abstand zum Kolben in der belasteten Stellung gehalten wird.

10. Baugruppe, umfassend eine Vorrichtung nach einem der vorhergehenden Ansprüche und eine Stange, deren eines Ende durch die distale Öffnung in den rohrförmigen Kanal des Laufs einführbar ist.

11. Baugruppe nach Anspruch 10, **gekennzeichnet durch** Mittel (17, 20, 21, 22) zum Greifen des Einsatzes, insbesondere an einem oberen freien Rand des Einsatzes, wobei die Stange (5, 6) zum Ausüben eines Schlags auf den Einsatz ausgelegt ist, wenn er durch die Greifmittel gegenüber dem Hohlabschnitt des Aufnahmeelements, insbesondere der Pfanne, gehalten wird.

12. Baugruppe nach einem der Ansprüche 10 oder 11, **dadurch gekennzeichnet, dass** sie einen Schlagkopf umfasst, der eine Oberfläche aufweist, die ausgelegt ist, mit zumindest einem Bereich des Einsatzes in Kontakt zu kommen, insbesondere der Innenfläche einer Ausnehmung des Einsatzes, und dass der Schlagkopf und die Beaufschlagungsstange drehfest miteinander verbunden sind, wobei insbesondere der Kopf an der Stange angeformt ist und die Schlagkopf-und Stangenanordnung relativ zu dem Mitteln zum Greifen des Einsatzes schwenkbar gelagert ist, insbesondere nach Art eines Kugelgelenks.

13. Baugruppe nach einem der Ansprüche 10 bis 12, **dadurch gekennzeichnet, dass** sie Anschlagmittel umfasst, die verhindern, dass die Stange über ein proximales Stück der Stange hinaus in den Lauf eindringt.

14. Baugruppe nach Anspruch 13, **dadurch gekennzeichnet, dass** das einen Anschlag bildenden Mittel aus einem aus der Stange seitlich herausragenden kreisförmigen Ring bestehen.

15. Anordnung, umfassend eine Baugruppe nach einem der Ansprüche 10 bis 14 und mindestens einen Einsatz (1), insbesondere aus Keramik, der ausgelegt ist, in eine Hüftgelenkspfanne eingesetzt zu werden.

## Claims

1. An impacting device for imparting a shock to a rod (6) or for "impacting" a rod (6) intended to transmit the shock to an insert element so as to insert the latter into a receiving element, for example an insert element, made in particular of ceramic material, into a cup itself intended to be received in the cotyloid cavity of a patient's hip, comprising a body (30) forming a barrel (31) defining an inner channel (35) having a longitudinal axis and leading to a distal end opening via which at least a proximal end of the rod can be inserted, impacting means intended to impart a shock having a predetermined force to the proximal end of the rod inserted into the channel (35) and gripping means so that the impacting device can be gripped by at least one of the surgeon's hands, **characterised in that** the gripping means are made in the form of a pistol grip (41) so that the surgeon can grasp the impacting device with the whole of his hand, i.e. with at least the four fingers of one hand in contact with the pistol grip, on just one and the same side, below the barrel.

2. The device according to Claim 1, **characterised in that** stop means are provided so that during the application of the shock to the rod by the impacting means, the body forming the barrel, under a force exerted by the user holding the impacting device by the pistol grip, also exerts pressure upon the rod in order to press it against the insert.

3. The device according to any of the preceding claims, **characterised in that** the arrangement is such that only an end section of the rod can penetrate into the channel, the stop means preventing the rod from penetrating beyond the section.

4. The device according to any of Claims 1 to 3, **characterised in that** the impacting means for imparting a shock with a pre-determined force to the rod comprise a plunger and a spring.

5. The device according to Claim 4, **characterised in that** means are provided for displacing the plunger against the compression of the spring so as to compress the spring.

6. The device according to any of the preceding claims, **characterised in that** means are provided that form a trigger for triggering the impacting means.

7. The device according to Claim 5, **characterised in that** the arrangement is such that the surgeon can trigger the impacting means by acting on a trigger with a finger of his hand, in particular the index finger, while holding the device by the gripping means, having his forearm on the one hand aligned with the back of the palm of his hand and on the other hand parallel to the longitudinal axis of the channel.

8. The device according to Claim 4, **characterised in that** means are provided for blocking the plunger in a charged position in which the spring is compressed, as are means, in particular in the form of a trigger, intended to release the plunger, the release of the plunger causing its instantaneous displacement by the decompression of the spring and its pulse action on the rod imparting to the latter a shock with pre-determined force.

9. The device according to Claim 4, **characterised in that** the spring and the plunger are received in a cavity proximal to the rod, the section of the rod introduced into the channel being maintained by the stop means a distance away from the plunger in the charged position.

10. An assembly comprising a device according to any of the preceding claims and a rod of which one end can be inserted into the tubular channel of the barrel via the distal opening.

11. The assembly according to Claim 10, **characterised by** insert gripping means (17, 20, 21, 22), in particular at the level of a free upper edge of the insert, the rod (5, 6) being intended to impact on the insert when the latter is held by the gripping means opposite the hollow part of the receiving element, in particular the cup.

12. The assembly according to either of Claims 10 or 11, **characterised in that** it comprises an impacting head that has a surface intended to come into contact with at least part of the insert, in particular the inner surface of a hollow of the insert, and the impacting head and the application rod are connected to one another so as to be joined in rotation, in particular the head emerges from the rod and the impacting head and rod assembly is mounted pivotably relative to the means for gripping the insert, in particular in the manner of a ball joint.

13. The assembly according to any of Claims 10 to 12, **characterised by** stop means that prevent the rod from penetrating into the barrel beyond a proximal section of the rod.

14. The assembly according to Claim 13, **characterised in that** the stop means are formed by a circular ring projecting laterally from the rod.

15. A kit comprising an assembly according to any of Claims 10 to 14 and at least one insert (1), in particular made of ceramic, intended to be inserted into a cotyloid cup.
